Europäisches Patentamt

(19) European Patent Office

Office européen des brevets

(11) Publication number: **0 131 465**

**B1**

(12) **EUROPEAN PATENT SPECIFICATION**

(45) Date of publication of patent specification: **09.03.88**

(21) Application number: **84304681.4**

(22) Date of filing: **09.07.84**

(51) Int. Cl.⁴: **B 01 J 27/22, C 07 C 1/04**

(54) **Iron on titania catalyst and its use for hydrocarbon synthesis.**

(30) Priority: **07.07.83 US 511651**
**07.07.83 US 511653**
**02.07.84 US 625169**
**02.07.84 US 625170**

(43) Date of publication of application:
**16.01.85 Bulletin 85/03**

(45) Publication of the grant of the patent:
**09.03.88 Bulletin 88/10**

(84) Designated Contracting States:
**DE GB NL**

(56) References cited:
**EP-A-0 071 770**
**DE-B-2 536 488**
**FR-A- 978 243**
**US-A-2 543 327**
**US-A-4 192 777**
**US-A-4 261 865**
**US-A-4 289 655**

(73) Proprietor: **Exxon Research and Engineering Company**
**P.O.Box 390 180 Park Avenue**
**Florham Park New Jersey 07932 (US)**

(72) Inventor: **Fiato, Rocco Anthony**
**275 Country Club Lane**
**Scotch Plains New Jersey 07076 (US)**
Inventor: **Kugler, Edwin Lee**
**R.D.2 P.O. Box 38B**
**Glen Gardner New Jersey 08826 (US)**

(74) Representative: **Somers, Harold Arnold et al**
**ESSO Engineering (Europe) Ltd. Patents & Licences Apex Tower High Street**
**New Malden Surrey KT3 4DJ (GB)**

Courier Press, Leamington Spa, England.

## Description

**Background of the invention**

The use of iron-titania mixtures as Fischer-Tropsch catalysts for converting mixtures of CO and $H_2$ to hydrocarbons is well-known to those skilled in the art. For example, U.S. Patent 2,543,327 discloses titania promoted iron oxide for Fischer-Tropsch synthesis wherein the iron oxide is in the form of naturally occurring magnetite and preferably as Alan Wood ore. In this disclosure a typical catalyst is shown as prepared by mixing about 13,600 grams of Alan Wood ore with 98 grams of titania and 216 grams of potassium carbonate used as a promoter. The ratio of hydrogen to carbon monoxide disclosed as being preferably at least 2/1 and the results show that the catalyst has relatively poor activity with a large selectivity towards the production of methane and very little selectivity towards the production of $C_2^+$ hydrocarbons. That is, the Fischer-Tropsch product was primarily methane. Similarly, British patent 1,512,743 also discloses a titania promoted, massive iron type of Fischer-Tropsch catalyst wherein ion oxide is mixed with titanium oxide, zinc oxide and potassium carbonate with the resulting mixture being sintered and then reduced for many hours at 500°C. Although this catalyst has relatively reasonable activity with regard to conversion of the CO and $H_2$ mixture, the product was primarily (i.e., about 73%) olefinic, unsaturated $C_2/C_4$ hydrocarbons and with only about 10% of $C_2/C_4$ saturated hydrocarbons or alkanes being produced, U.S. Patent 4,192,777 and 4,154,751 while directed towards the use of potassium promoted Group VIII metal cluster catalysts in Fischer-Tropsch synthesis reactions, suggest that iron supported on titania would be useful Fischer-Tropsch catalysts but do not disclose the preparation of same. In their examples, they disclose iron on various supports other than titania with the amount of iron on the support generally being less than about 5 percent. U.S. Patent 4,261,865 discloses an iron titanate-alkali metal hydroxide catalyst for preparing alpha-olefins from mixtures of CO and $H_2$. That is, the catalyst is not iron supported on titania along with an alkali metal hydroxide but rather an iron titanate compound.

Another example of a titania-promoted massive iron catalyst for Fischer-Tropsch synthesis may be found in the Volume 17, No. 3—4 React. Kinet. Catal. Lett., pages 373—378, (1981) titled "Hydrocondensation of $CO_2$ (CO) Over Supported Iron Catalysts". This article discloses an iron oxide, titania, alumina, copper oxide catalyst promoted with potassium. Similarly, in European patent application EP 0 071770 A2 Fischer-Tropsch catalysts are disclosed which include iron titania catalysts wherein the iron to titania ratio can be greater than 1/10. The actual iron-titania catalyst is not an iron supported on titania catalyst but an iron/titania catalyst produced by a coprecipitation technique wherein the active iron catalytic component is distributed throughout a titanium oxide matrix. Thus, the resulting catalyst was not iron supported on titania but rather a bulk phase iron/titania mixture which, when used for Fischer-Tropsch synthesis, produced predominantly olefins. The amount of olefins produced was generally greater than about 80% of the total hydrocarbon product.

With regard to iron/titania catalysts for Fischer-Tropsch wherein the iron is supported on titania, a 1982 article by Vannice, *Titania-Supported Metals as CO Hydrogenation Catalysts*, J. Catalysis, v. 74, p. 199—202 (1982), discloses the use of an iron/titania catalyst for Fischer-Tropsch synthesis wherein the amount of iron, calculated as metallic iron, is 5 percent of the iron/titania composite and the catalyst shows extremely little activity for Fischer-Tropsch synthesis.

An article by Reymond et al, *Influence of the Support or of an Additive on the Catalytic Activity in The Hydrocondensation of Carbon Monoxide by Iron Catalysts* in "Metal-Support and Metal-Additive Effects in Catalysis", B. Imelik et al. (Eds), Elsevier, Netherlands, p. 337—348 (1982), also discloses the use of iron/titania Fischer-Tropsch catalysts wherein the iron is supported on the titania. The iron/titania catalysts disclosed contain about 9.5 weight percent iron on titania and the activity of the resulting catalysts is presented as a function of the activation pretreatment of the iron/titania catalyst precursor. Thus, it was disclosed that if the precursor was pretreated in either helium or hydrogen at 250°C there was relatively little activity for Fischer-Tropsch synthesis. Similarly, another composite treated in hydrogen for 15 hours at 500°C showed no activity whatsoever. It is important to note that the catalytic activity was expressed only as a function of methane production using a 9/1 mole ratio of $H_2/CO$ at one atmosphere pressure and a reaction temperature of 250°C.

The present invention provides a catalyst comprising a mixture of iron carbide and ilmenite supported on titania.

In another aspect, the invention provides a process for producing a catalyst comprising a mixture of ilmenite and iron carbide supported on titania useful for producing substantially alkane hydrocarbons from mixtures of CO and $H_2$ comprising the steps of:

(a) depositing iron on a titania support material from a solution of iron precursor compound in an amount such that the final catalyst will contain supported iron in an amount of at least 2 milligrams of iron, calculated as $Fe_2O_3$, per square meter of titania support surface;

(b) calcining the iron precursor compound supported on titania produced in step (a) at an elevated temperature of at least 120°C, (e.g. in the range of from about 120 to 500°C) to decompose said iron precursor material and convert at least a portion of said supported iron to $Fe_2O_3$;

(c) contacting said calcined composite formed in step (b) with hydrogen at a temperature in the range of from 300 to 500°C to convert at least a portion of said supported iron to a reduced composite; and

(d) contacting said reduced composite formed in (c) with CO at an elevated temperature of at least 200°C for a time sufficient to form said catalyst.

In yet another aspect, the invention provides a process for producing hydrocarbons, including alkane hydrocarbons, from a gaseous mixture of CO and $H_2$ comprising contacting said mixture, at a temperature ranging from 200 to 350°C to convert at least a portion of said feed to alkane hydrocarbons, with a catalyst comprising a mixhure of iron carbide and ilmenite supported on titania wherein the amount of said supported iron present in said supported iron carbide and ilmenite, calculated as $Fe_2O_3$, is at least $2 \times 10^{-3}$ grams per square meter of titania support surface.

It has now been discovered that substantially $C_2^+$ alkane hydrocarbons can be produced from mixtures of CO and $H_2$ using a catalyst comprising iron carbide and ilmenite supported on titania. Those skilled in the art know that ilmenite is an iron titanate having the formula $FeTiO_3$. The ratio of the iron present in said supported iron carbide and ilmenite, calculated as $Fe_2O_3$, to the surface area of the titania support will generally range from about 2 to 25 milligrams per square meter. By "substantially $C_2^+$ alkane hydrocarbons" is meant that more than about 50 wt.% of the hydrocarbon products, including methane, are alkane $C_2^+$ hydrocarbons. In a preferred embodiment the catalyst will be pretreated with CO at elevated temperature prior to use.

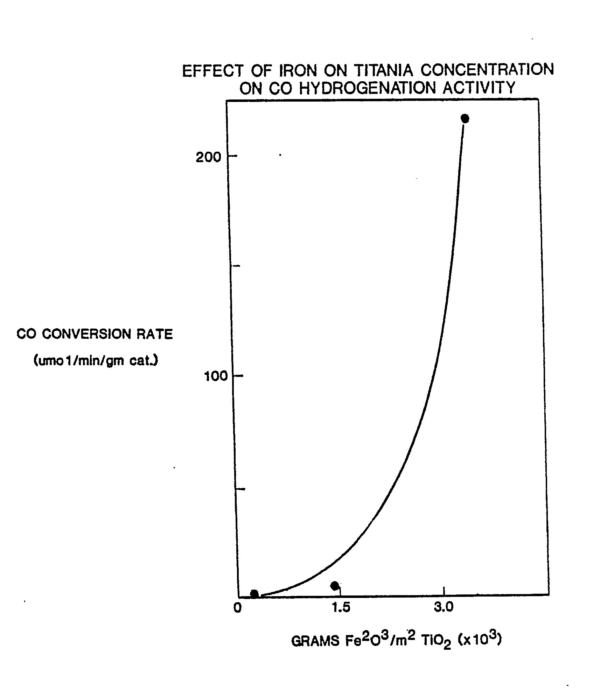

Brief description of the drawing

The figure is a plot of CO conversion rate as a function of the iron loading level of a catalyst of this invention.

Detailed description

It is essential to this invention that a mixture of the iron carbide and ilmenite is supported on and not merely mixed with the titania support. The iron loading on the titania support must be sufficient to form a mixture of both iron carbide and ilmenite. In general, it has been found that this will occur if the iron loading, calculated as $Fe_2O_3$, is at least about 2 milligrams per square meter of titania support surface. It has been found that if the titania doesn't support at least about 2 milligrams of iron, calculated as $Fe_2O_3$ per $m^2$ of titania support surface, the catalyst will possess little or no activity for conversion of mixtures of CO and $H_2$ to hydrocarbons. On the other hand, it has been found that selectivity of the catalyst to alkane formation rapidly decreases if more than about 25 milligrams of iron, calculated as $Fe_2O_3$, per $m^2$ of $TiO_2$ support surface is loaded onto the titania support. Preferably, the amount of iron present in the iron carbide and ilmenite mixture on the titania support will range from about 2.8 to 8.3 milligrams, calculated as $Fe_2O_3$, per $m^2$ of titania support surface.

The catalyst will be prepared by depositing a suitable iron precursor component onto the titania support from a precursor solution using any of the well known techniques such as incipient wetness, multiple impregnation, pore-filling etc., the choice being left to the convenience of the practitioner. As has heretofore been stated, it is important for the iron precursor to be deposited onto the titania support as opposed to other methods for catalyst preparation such as co-precipitation or physical mixtures. After impregnation, the impregnate is dried to remove excess solvent and/or water therefrom. The dry impregnate can then be converted to a catalyst of this invention employing a number of different methods. In one method, the impregnate will be converted directly to a catalyst of this invention by contacting same with a CO-containing reducing gas, preferably a reducing gas containing a mixture of CO and $H_2$. Thus, it will be appreciated by those skilled in the art that the catalyst of this invention can be formed from the impregnate in-situ in a Fischer-Tropsch hydrocarbon synthesis reactor. However, it is preferred to employ a sequential treatment of first contacting the dry impregnate with an $H_2$-containing reducing gas that does not contain CO to reduce the impregnate, followed by contacting the reduced impregnate with CO or a CO-containing gas such as a mixture of CO and $H_2$ to form the catalyst of this invention. As a practical matter, it may be commercially advantageous to form the catalyst of this invention by subjecting the impregnate to calcining to convert the supported iron precursor component to iron oxide, followed by subsequent reduction and formation of the catalyst of this invention.

Promoter metals such as potassium or other alkali metals may be added via impregnation, etc, before the composite is contacted with a reducing atmosphere and/or CO-containing gas to form the catalyst of this invention. In general, the amount of promoter metal present will range from about 0.5 to 5 wt.% based on the amount of iron (calculated as $Fe_2O_3$) supported on the titania.

If one desires to obtain a catalyst of this invention via a supported iron oxide route, then the dry impregnate will be calcined in air or other suitable oxidizing atmosphere at a temperature of from about 120 to 300°C for a time sufficient to convert the supported iron precursor component to iron oxide. After the iron/titania impregnate has been calcined to convert the supported iron precursor compound to iron oxide, the iron oxide/titania composite, with or without one or more promoter metals, is preferably reduced in a hydrogen-containing, net-reducing atmosphere at a temperature broadly ranging from about 300—500°C for a time sufficient to convert the iron oxide to metallic iron. It has been found that if one tries to reduce the iron oxide/titania composite at a temperature below about 300°C (i.e., 250°C), the catalyst of this invention will not subsequently be formed.

Irrespective of the route one employs to form a catalyst of this invention, whether by reduction followed by contacting with CO, direct formation of the catalyst or through the supported iron oxide route, it is important not to contact the composite with a reducing gas at temperatures above about 500°C. Reduction temperatures exceeding about 500°C will produce a catalyst which exhibits relatively low CO hydrogenation activity with less than 50% of the $C_2^+$ hydrocarbons produced being alkanes. Further, even at a 500°C reduction temperature a less effective catalyst will be produced if the reduction occurs for too long a time, i.e., about ten hours or more. Thus it will be appreciated that the temperature range for reducing the composite cannot be critically quantified with any degree of precision, inasmuch as there exists a time-temperature continuum for proper reduction.

In a preferred embodiment of this invention, the catalyst composite will first be reduced, followed by contacting with CO at temperatures ranging from about 200 to 500°C and preferably 300 to 400°C for a time sufficient to form a catalyst comprising a mixture of ilmenite and iron carbide supported on titania. It has been found that a CO treatment following hydrogen reduction dramatically improves the activity of the catalyst for CO conversion with only slight changes in product selectivity. A mixture of ilmenite and iron carbide on the titania support will also be achieved by treating the calcined iron/titania composite with a mixture of CO and $H_2$, but it is preferred to use the sequential treatment comprising hydrogen reduction followed by CO treatment. Further, when using this sequential treatment to produce a catalsyt of this invention, it is preferred that the temperature used for the CO treatment be lower than that used for the hydrogen reduction. Thus, in general the CO treatment will occur at a temperature of about 100 to 200°C lower than the temperature used for the hydrogen reduction.

It has also been discovered that, if a catalyst composite of this invention has been prepared by hydrogen reduction and then contacted in-situ, in a reactor, with a feedstream comprising a mixture of CO and $H_2$ to form a catalyst of this invention, the activity of the so-formed catalyst will be substantially increased by reducing or eliminating the hydrogen content of the feedstream, raising the temperature in the reactor an additional 50 to 150°C for a short period of time (i.e., 3—5 hours), followed by reestablishing the original reaction conditions.

Predominantly $C_2^+$ alkane hydrocarbons are produced from mixtures of CO and $H_2$ by contacting said mixtures with the catalyst of this invention at temperatures ranging from about 200 to 350°C and preferably from about 250—320°C. The (gauge) reaction pressure will generally range from about 100—500 psig (689.5 to 3447.5 kPa) and more preferably from about 150—300 psig (1034 to 2069 kPa), although pressures outside this range may be used, if desired. However, if one goes too low in pressure (i.e., <50 psig, <344.7 kPa gauge), catalyst activity will be greatly reduced and methane production will predominate. Upper pressure limits will generally be dictated by economic considerations. The $H_2$/CO mole ratio in the reaction zone will generally range from about 1/2 to 3/1, preferably from about 1/2 to 2/1 and still more preferably from about 1/2 to 1/1.

The invention will be more readily understood by reference to the following examples.

Examples
Example 1

In this experiment a number of iron supported on titania catalysts were prepared by impregnating, at room temperature, a titania powder (Degussa P-25) with aqueous solutions of ammonium trisoxalato ferrate containing different amounts of the iron salt. The resulting impregnates were dried in air. After drying, each impregnate was ground to a powder and calcined in air for at least one hour at 200°C to form an iron oxide/titania composite. A 1—2 cm³ sample of each composite was loaded into a 3/8 inch 9.525 mm O.D. (outside diameter) stainless steel tube reactor. The reactor was flushed with hydrogen at room temperature and atmospheric pressure. The reactor temperature was then brought up to 450°C in flowing hydrogen (90 cm³/min) and maintained at these conditions for 1—2 hours. After this, the reactor was cooled to a temperature of 300°C and the (gauge) pressure increased to 150 psig (1034 kPa). The hydrogen was then replaced with a 3/1 mole mixture of $H_2$/CO at a flow rate (standard hourly velocity) of 3600 v/v/hr. The exit gas from the reactor was fed into a gas chromatograph for on-line analysis of $C_1$—$C_{15}$ hydrocarbons, CO, $CO_2$, and $N_2$.

The results of this experiment are plotted in the Figure in terms of CO conversion rate as a function as the iron loading level on the catalyst calculated as grams of $Fe_2O_3$ per m² of $TiO_2$ surface area. These results dramatically illustrate an unexpected, minimum critical iron loading level for Fischer-Tropsch activity of about $2 \times 10^{-3}$ grams of $Fe_2O_3$ per m² of titania.

Example 2

Another catalyst of this invention was prepared, consisting of $2 \times 10^{-3}$ grams of iron, calcupated as $Fe_2O_3$, per square meter of titania support. This was prepared by mixing an aqueous solution of ferric nitrate with a titania slurry (Degussa P-25), with stirring, for an hour at 25°C. The mixture was then heated to 120°C for a three hour period at a pressure of 45 mmHg (5.9985 kPa) pressure to remove the solvent and form a solid impregnate. The impregnate was then ground to a powder and dried overnight at 120°C under vacuum, followed by drying in air overnight at 130—150°C. The dried, calcined, powdered composite was then pelletized at 5000—15000 psi (34475 to 103425 kPa), crushed and sieved to 20—80 Tyler mesh (0.84 to 0.177 mm) particles.

8.8 cm³ of the calcined catalyst composite was loaded into a 1/2 inch (12.7 mm) O.D. stainless steel

4

tubular reactor which was then purged with hydrogen at 50°C and atmospheric pressure. The (gauge) pressure was then raised to 100 psig (689.5 kPa) and a 9/1 mole mixture of $H_2/N_2$ introduced into ths reactor at a rate of 100 cm$^3$/min. The temperature in the reactor was then increased to 500°C at a rate of 6°C/min. and was maintained at these conditions for five hours to form the catalysts. The $H_2/N_2$ stream was then replaced with a Fischer-Tropsch feedstream consisting of a 1/1 mole ratio of $CO/H_2$ diluted with 10 volume percent nitrogen. The reactor (gauge) pressure had been raised to 300 psig (2068 kPa) and the temperature reduced to 270°C before the gas feed was introduced at a rate (standard hourly space velocity) of 500 v/v/hr. As in Example 1, the reactor effluent was fed into a gas chromatograph.

The results of this experiment are set forth in Table 1 and show that 57.2 percent of the hdyrocarbon products were alkanes with less than 25 percent methane production.

Example 3

The experiment of Example 2 was repeated with the exception that the calcined catalyst composite was sequentially treated first with the 9/1 mole mixture of $H_2/N_2$ for five hours at 500°C and then with a 9/1 mole ratio mixture of $CO/N_2$ for five hours at 350°C. The results of this experiment, also shown in Table 1, demonstrate the beneficial affects of the sequential hydrogen CO treatment in terms of increased CO conversion, higher alkane yield, and greater $C_5^+$ alkane yield.

Example 4

Another catalyst of this invention was prepared containing $4.2 \times 10^{-3}$ grams of iron, calculated as $Fe_2O_3$, per m$^2$ of titania support by adding a solution of 38.16 grams of ammonium trisoxalato ferrate in 60 milliliters of distilled water to 44.8 grams of titania (Degussa P-25). The resulting mixture was dried at 65°C in air for three days. The resulting impregnated solid was ground to powder and heated at 200°C for six hours to decompose the iron complex and calcine the impregnate. The resulting powder was subsequently cooled to room temperature and impregnated with 0.157 grams of potassium carbonate dissolved in 10 ml water. The mixture was dried in air at 120°C for one hour to produce a potassium promoted composite wherein the amount of potassium was 4.28 percent based on the iron content, calculated as $Fe_2O_3$, of the calcined composite.

The potassium containing composite was then pelletized, crushed and sieved to 20—80 Tyler mesh (0.84—0.177 mm) particles, 8.8 cm$^3$ of which were loading into a 1/2 inch (12.7 mm) stainless steel reactor and treated using the procedure given in Example 2. The results, shown in Table 2, illustrate less than about 5 percent olefin production.

Example 5

Another catalyst of this invention was prepared following the procedure of Example 2 to form a calcined composite which was pelletized, crushed and sieved wherein the catalyst contained $8.3 \times 10^{-3}$ grams of iron, calculated as $Fe_2O_3$, per square meter of titania support. This composite was reduced with a mixture of 20 percent hydrogen in helium for two hours at 450°C and then cooled to 25°C in the flowing gas. When room temperature was achieved, the hydrogen flow was stopped and oxygen was introduced into the flowing helium at a 2 percent level in order to passivate the reduced composite. X-ray diffraction patterns of this hydrogen reduced material showed $TiO_2$ (both anatase and rutile), $FeTiO_3$ (ilmenite) and Fe° (metallic iron). This same reduced sample was then treated with CO for one hour at 350°C and cooled to room temperature in the same gas. After this, the sample was flushed with helium and then passivated with 3 percent oxygen in helium and the X-ray diffraction pattern measured again. It was found that the CO treatment as used in Example 3, had no effect on the X-ray powder diffraction pattern of the titania and ilmenite, but caused the X-ray diffraction pattern of metallic iron to disapear. Also, a broadened pattern of iron carbide $Fe_5C_2$ appeared after the CO treatment, indicating that CO converted the metallic iron to small particles of iron carbide.

## TABLE 1

| Catalyst Treatment | $H_2$ | $H_2$, CO |
|---|---|---|
| Temperature, °C | 270 | 270 |
| % CO Conversion | 26.8 | 59.5 |
| Wt. % Selectivity | | |
| $CH_4$ | 21.0 | 13.8 |
| $C_2^=$ | 0.8 | 1.0 |
| $C_2^\circ$ | 16.2 | 13.5 |
| $C_3^=$ | 18.6 | 12.1 |
| $C_3^\circ$ | 11.8 | 5.4 |
| $C_4^=$ | 2.4 | 4.1 |
| $C_4^\circ$ | 6.2 | 5.3 |
| $C_5^+$ | 23.0 | 44.8 |
| $C_2^=\!-\!C_4^=/C_2^\circ\!-\!C_4^\circ$ | 0.64 | 0.71 |

Conditions: 2 MPa, 500 v/v/hr, 1:1 $H_2$:CO, $H_2$ pretreatment at 500°C, CO pretreatment at 350°C. Composite $C_5^+$ determined by $N_2$ internal standard method.

## TABLE 2
### Potassium promoted catalyst

| | |
|---|---|
| % CO Conversion | 89.2 |
| Wt. % Selectivity | |
| $CH_4$ | 18.9 |
| $C_2^=$ | 0.6 |
| $C_2^\circ$ | 18.2 |
| $C_3^=$ | 2.2 |
| $C_3^\circ$ | 17.8 |
| $C_4^=$ | 1.4 |
| $C_4^\circ$ | 11.1 |
| $C_5^+$ | 29.8 |
| $C_2^=\!-\!C_4^=$ | 4.2 |
| $C_2^\circ\!-\!C_4^\circ$ | 47.1 |

Conditions: 290°C, 2 MPa, 500 v/v/hr, 1:1 $H_2$:CO, pretreatment with $H_2$ at 500°C for 5 hr ($\neq$10 hr) and CO at 350°C for 5 hr.
Composite $C_5^+$ determined by $N_2$ internal standard method.

The present invention, in another aspect, provides a process for producing a catalyst comprising a mixture of ilmenite and iron carbide supported on titania substantially as herein described.

The present invention, in another aspect provides a process for producing hydrocarbons including alkane hydrocarbons from a gaseous mixture of CO and $H_2$ substantially as herein described.

In another aspect, the invention provides a process for producing predominantly alkane hydrocarbons from a gaseous feed mixture of $H_2$ and CO comprising contacting said feed, at a temperature in the range of from about 200 to 350°C and for a time sufficient to convert with at least a portion of said feed to alkane hydrocarbons, with a catalyst comprising a mixture of iron carbide and ilmenite supported on titania, wherein the amount of iron present in said supported iron carbide and ilmenite, calculated as $Fe_2O_3$, is at least about $2\times10^{-3}$ grams of iron per square meter of titania support surface, said catalyst having been formed by depositing an iron precursor compound on said titania support to form an iron/titania composite, calcining said composite to decompose said precursor compound and convert at least a portion thereof to iron oxide, followed by reducing said iron oxide/titania composite by first contacting same with hydrogen at a temperature of at least about 300°C for a time sufficient to form a reduced composite and then contacting said reduced composite with CO at a temperature of at least about 200°C for a time sufficient to form said catalyst.

In another aspect, the present invention provides a process for improving the activity of a Fischer-Tropsch catalyst present in a Fischer-Tropsch reaction zone wherein said catalyst comprises a mixture of iron carbide and ilmenite supported on a titania support, said process comprising reducing or eliminating the hydrogen content of the Fishcer-Tropsch feedstream, raising the temperature in said reactor by from about 50 to 150°C for from about 1 to 6 hours and then lowering the temperature back to reaction temperature and by re-establishing the hydrogen content of the feedstream.

## Claims

1. A catalyst comprising a mixture of iron carbide and ilmenite supported on titania.

2. A catalyst according to claim 1 useful for producing $C_2{}^+$ alkane hydrocarbons from mixtures of CO and $H_2$ comprising a mixture of iron carbide and ilmenite supported on a titania support wherein the amount of iron present in said supported iron carbide and ilmenite, calculated as $Fe_2O_3$, ranges between about 2 to 25 milligrams per square meter of titania support surface.

3. The catalyst of claim 1 or claim 2 containing one or more alkali promoter metals wherein said promoter metal is present on the catalyst in an amount ranging from 0.5 to 5 wt.% based on the amount of supported iron calculated as $Fe_2O_3$.

4. The catalyst of any one of claims 1 to 3 which has been reduced by contact with hydrogen, prior to use.

5. The catalyst of any one of claims 1 to 4 which has been reduced by contact with CO, prior to use.

6. The catalyst of claim 4 or claim 5 wherein said reduction is effected at a temperature in the range of from about 200 to 500°C.

7. A process for producing a catalyst comprising a mixture of ilmenite and iron carbide supported on titania useful for producing substantially alkane hydrocarbons from mixtures of CO and $H_2$ comprising the steps of:

(a) depositing iron on a titania support material from a solution of iron precursor compound in an amount such that the final catalyst will contain supported iron in an amount of at least 2 milligrams of iron, calculated as $Fe_2O_3$, per square meter of titania support surface;

(b) calcining the iron precursor compound supported on titania produced in step (a) at an elevated temperature of at least 120°C, to decompose said iron precursor material and convert at least a portion of said supported iron to $Fe_2O_3$;

(c) contacting said calcined composite formed in step (b) with hydrogen at a temperature in the range of from 300 to 500°C to convert at least a portion of said supported iron to a reduced composite; and

(d) contacting said reduced composite formed in (c) with CO at an elevated temperature of at least 200°C to form said catalyst.

8. The process of claim 7 wherein said reduced composite is contacted with CO at a temperature in the range of from 200 to 500°C prior to use.

9. The process of claim 8 in which said CO contacting is effected at a temperature of from 100 to 200°C lower than the temperature at which the calcined composite is contacted with hydrogen.

10. A process for producing hydrocarbons, including alkane hydrocarbons, from a gaseous mixture of CO and $H_2$ comprising contacting said mixture, at a temperature ranging from 200 to 350°C to convert at least a portion of said feed to alkane hydrocarbons, with a catalyst comprising a mixture of iron carbide and ilmenite supported on titania wherein the amount of said supported iron present in said supported iron carbide and ilmenite, calculated as $Fe_2O_3$, is at least $2\times10^{-3}$ grams per square meter of titania support surface.

11. The process of claim 10 wherein the amount of supported iron present in said supported iron carbide and ilmenite, calculated as $Fe_2O_3$, is in the range of from 2 to 25 milligrams of iron per square meter of titania support surface.

## Patentansprüche

1. Katalysator, der eine Mischung aus Eisencarbid und Ilmenit auf Titandioxid als Träger enthält.

2. Katalysator gemäß Anspruch 1, der zur Darstellung von $C_2$-Alkanen aus Mischungen von CO und $H_2$ geeignet ist und eine Mischung aus Eisencarbid und Ilmenit auf einem Titandioxidträger enthält, wobei die vorhandene Menge an Eisen in besagtem Eisencarbid und Ilmenit auf dem Träger, als $Fe_2O_3$ berechnet, im Bereich zwischen 2 und 25 mg pro $m^2$ der Oberfläche des Titandioxidträgers liegt.

3. Katalysator nach Anspruch 1 oder 2, der einen oder mehrere Alkalimetallpromotoren enthält, wobei das besagte Promotormetall in einer Menge von 0,5 bis 5 Gew.% im Katalysator vorliegt, bezogen auf die Menge an Eisen (berechnet als $Fe_2O_3$) auf dem Träger.

4. Katalysator nach einem der Ansprüche 1 bis 3, der vor seiner Verwendung durch Kontakt mit Wasserstoff reduziert wurde.

5. Katalysator nach einem der Ansprüche 1 bis 4, der vor der Verwendung durch Kontakt mit CO reduziert wurde.

6. Katalysator nach Anspruch 4 oder 5, bei welchem die besagte Reduzierung in einem Temperaturbereich von ungefähr 200 bis 500°C erfolgt ist.

7. Verfahren zur Herstellung eines eine Mischung aus Ilmenit und Eisencarbid auf Titandioxid als Träger enthaltenden Katalysators, der bei der Herstellung von im wesentlichen Alkanen aus Mischungen aus CO und $H_2$ von Nutzen ist, wobei besagtes Verfahren durch folgende Schritte gekennzeichnet ist:

a) Niederschlagen von Eisen auf das Titandioxidträgermaterial aus einer Lösung einer als Vorläufer dienenden Eisenverbindung in einer solchen Menge, daß der fertige Katalysator Eisen auf dem Träger in einer Menge von zumindest 2 mg Eisen, als $Fe_2O_3$ berechnet, pro $m^2$ der Oberfläche des Titandioxidträgers enthält;

b) Calcinierung der Eisenvorläuferverbindung auf dem Titandioxid als Träger, hergestellt nach Schritt (a), bei erhöhter Temperatur von zumindest 120°C, um besagte Eisenvorläuferverbindung zu zersetzen und zumindest einen Teil des Eisens auf dem Träger in $Fe_2O_3$ umzuwandeln;

c) Kontaktieren besagter calcinierter Mischung, die nach Schritt b) erhalten wird, mit Wasserstoff bei einer Temperatur im Bereich von 300 bis 500°C, um zumindest einen Teil des Eisens auf dem Träger in eine reduzierte Mischung umzuwandeln; und

d) Kontaktieren besagter reduzierter Mischung, die in Schritt c) gebildet wird, mit CO bei einer erhöhten Temperatur von zumindest 200°C, um besagten Katalysator zu bilden.

8. Verfahren nach Anspruch 7, in welchem besagte reduzierte Mischung vor der Verwendung bei einer Temperatur im Bereich von 200 bis 500°C mit CO in Berührung gebracht wird.

9. Verfahren nach Anspruch 8, in welchem besagter CO-Kontakt bei einer Temperatur erfolgt, die 100 bis 200°C tiefer ist als die Temperatur, bei welcher die calcinierte Mischung mit Wasserstoff in Berührung gebracht wird.

10. Verfahren zur Herstellung von Kohlenwasserstoffen einschließlich von Alkanen aus gasförmigen Mischungen von CO und $H_2$, gekennzeichnet durch das Kontaktieren besagter Mischung mit einem Katalysator bei einer Temperatur von 200 bis 350°C, um zumindest einen Teil besagter Ausgangsstoffe in Alkane umzuwandeln, wobei der Katalysator eine Mischung aus Eisencarbid und Ilmenit auf Titandioxid als Träger enthält, wobei die vorhandene Menge an Eisen, als $Fe_2O_3$ berechnet, in besagtem Eisencarbid und Ilmenit auf dem Träger zumindest $2 \times 10^{-3}$ g pro $m^2$ der Oberfläche des Titandioxidträgers beträgt.

11. Verfahren nach Anspruch 10, in welchem die Menge des vorhandenen Eisens in besagtem Eisencarbid und Ilmenit auf dem Träger, als $Fe_2O_3$ berechnet, im Bereich von 2 bis 25 mg Eisen pro $m^2$ der Oberlfäche des Titandioxidträgers beträgt.

**Revendications**

1. Un catalyseur comprenant un mélange de carbure de fer et d'ilménite déposé sur support de dioxyde de titane.

2. Un catalyseur selon la revendication 1, utile pour la production d'hydrocarbures alcaniques en $C_2^+$ à partir de mélanges de CO et d'$H_2$, comprenant un mélange de carbure de fer et d'ilménite déposé sur un support de dioxyde de titane, dans lequel la quantité de fer présente dans lesdits carbure de fer et ilménite sur support, calculée en $Fe_2O_3$, se situe entre environ 2 et 25 milligrammes par mètre carré de surface du support de dioxyde de titane.

3. Catalyseur selon la revendication 1 ou 2, contenant un ou plusieurs métaux activateurs alcalins, dans lequel le métal activateur est présent sur le catalyseur en une quantité s'étendant de 0,5 à 5% en poids par rapport à la quantité de fer sur support, calculée en $Fe_2O_3$.

4. Catalyseur selon l'une quelconque des revendications 1 à 3 qui a été réduit par mise en contact avec de l'hydrogène avant emploi.

5. Catalyseur selon l'une quelconque des revendications 1 à 4 qui a été réduit par mise en contact avec CO avant emploi.

6. Catalyseur selon la revendication 4 ou 5, dans lequel la réduction est réalisée à une température dans la gamme d'environ 200 à 500°C.

7. Un procédé de fabrication d'un catalyseur composé d'un mélange d'ilménite et de carbure de fer sur support de dioxyde de titane, destiné à la production d'hydrocarbures essentiellement alcaniques à partir de mélanges de CO et d'$H_2$, comprenant les étapes:

a) de dépôt de fer sur un support de dioxyde de titane à partir d'une solution d'un composé précurseur du fer en une quantité telle que le catalyseur final contienne le fer sur support à une dose d'au moins 2 milligrammes de fer, calculée en $Fe_2O_3$, par mètre carré de surface du support de dioxyde de titane;

b) de calcination du composé précurseur du fer sur support de dioxyde de titane produit dans l'étape (a) à une température élevée d'au moins 120°C en vue de la décomposition dudit composé précurseur de fer et de conversion d'au moins une partie dudit fer sur support en $Fe_2O_3$;

c) de mise en contact dudit composite calciné formé dans l'étape (b) avec de l'hydrogène à une température dans la gamme de 300 à 500°C en vue de la conversion d'au moins une partie dudit fer sur support en un composite réduit, et

d) de mise en contact dudit composite de fer formé dans (c) avec CO à une température élevée d'au moins 200°C pour former ledit catalyseur.

8. Procédé selon la revendication 7, dans lequel ledit composite réduit est mis en contact avec CO à une température dans la gamme de 200 à 500°C avant emploi.

9. Procédé selon la revendication 8, dans lequel le contact avec CO a lieu à une température de 100 à 200°C inférieure à la température à laquelle le composite calciné est mis en contact avec l'hydrogène.

10. Un procédé pour la production d'hydrocarbures, notamment d'hydrocarbures alcaniques, à partir d'un mélange gazeux de CO et de $H_2$, consistant à mettre ledit mélange en contact, à une température s'étendant de 200 à 350°C pour convertir au moins une partie de ladite charge d'alimentation en hydrocarbures alcaniques, avec un catalyseur composé d'un mélange de carbure de fer et d'ilménite sur support de dioxyde de titane dans lequel la quantité de fer supporté présente dans le carbure de fer et l'ilménite sur support, calculée en $Fe_2O_3$, est d'au moins $2\times10^{-3}$ grammes par mètre carré de surface du support de dioxyde de titane.

11. Procédé selon la revendication 10, dans lequel la quantité de fer sur support présente dans le carbure de fer et l'ilménite sur support, calculée en $Fe_2O_3$, se situe entre 2 et 25 milligrammes de fer par mètre carré de surface du support de dioxyde de titane.

EFFECT OF IRON ON TITANIA CONCENTRATION
ON CO HYDROGENATION ACTIVITY

CO CONVERSION RATE

(umo 1/min/gm cat.)

200

100

0        1.5        3.0

GRAMS $Fe^2O^3/m^2$ $TIO_2$ $(x10^3)$

1